# EUROPEAN PATENT APPLICATION

(11) **EP 0 619 141 A1**
(43) Date of publication of application: **12.10.1994**
(21) Application number: 94104471.1
(22) Date of filing: 22.03.1994
(51) Int. Cl.: B01J 21/08, B01J 37/00, B01J 37/03

(54) **Selective inorganic catalysts in the form of molecular imprints in cavities**

(30) Priority: 25.03.1993 DE 4309660
(71) Applicant: Studiengesellschaft Kohle mbH, D-45470 Mülheim (DE)
(72) Inventor: Maier, Wilhelm F., D-45289 Essen (DE)
(74) Representative: von Kreisler, Alek, Dipl.-Chem.

(57) **Abstract**

The present invention relates to a process for producing inorganic solids as catalysts which contain imprints of molecules in the form of cavities, which molecules in their three-dimensional structures conform to intermediates or transition states of chemical reactions to be catalyzed, characterized in that inorganic or organometal monomer units are polymerized in the presence of the molecules to be imprinted, wherein the molecules to be imprinted have been chemically bonded to anchor groups (mo nomer units) and are admixed prior to or in the beginning of the polymerization process and the imprinted molecules are subsequently removed after the polymerization, to the resulting catalysts and to the use thereof.

## Description

Many chemical products are prepared by using a homogeneous or heterogeneous catalyst. Enzymes, i.e. catalysts of the biological systems, consisting of polyproteins operate under mild conditions with a high activity and selectivity. Many commercially important heterogeneous catalysts may be described as chemically, thermally and mechanically stable inorganic support materials (metal oxides in most cases) having a large total surface area and comprising a high concentration of finely distributed active sites on their surfaces (Catalyst Handbook, Editor: M.V. Twiggs, Second Edition, Wolfe Publishing, Cleveland 1989). In comparison to enzymes, these catalysts are capable of catalyzing a substantially broader spectrum of chemical reactions. The particular stability thereof allows them to be used at high temperatures (high space-time yields) with high conversion figures, frequently a relatively simple regenerability and an easy catalyst recovery. With respect to all of these properties commercial catalysts are mostly superior to the enzyme catalysts. However, so far unattainable by such commercial catalysts are the high selectivity and substrate specificity of enzymes. An important contribution to heterogeneous catalysis would be afforded by a development of heterogenous catalysts that are capable of combining both of the properties of the two systems mentioned - the high activity and stability of the commercial catalysts and the high selectivity of the natural catalysts.

The specificity is provided by the spatial structure of the active site in the enzyme. Enzymes accelerate reactions by stabilizing the transition state of the reaction. This means that the enzyme has a higher affinity to the transition state or to a molecule analogous thereto (transition state analogue) than to the starting materials or final products of a reaction {L. Pauling, *Chem. Eng. News* **24** (1946) 1375}. This postulated interpretation of the catalytic activity of enzymes has been confirmed long ago by X-ray structure analyses of the bonding sites of various enzymes. The affinity of enzymes to transition state analogues or structurally similar compounds is utilized to an increasing degree for blocking enzymes (enzyme inhibitors) {P.A. Bartlett, C.K. Marlowe, *Biochemistry* **22** (1983) 4618}. Although the use of enzymes in chemistry continuously increases, it is limited by the availability of these evolutionary catalysts for individual reactions. So far there has not been any possibility of purposefully developing enzymes within a reasonable margin of time for non-natural chemical reactions.

However, it was shown that antibodies produced against protein-bound transition state analogues can be used as enzyme-like catalysts {Lerner, Benkovic, Schultz, *Science* **252** (1991) 659}.

The fact that selective cavities can be produced not only in antibodies, but also in organic polymers, has already been made use of in chromatographic separations. Thus, chiral pockets were generated by the polymerization of organic monomers in the presence of suitable templates, which pockets could be employed for separating racemates in chromatography {G. Wulff, M. Minarik, S. Schauhoff, *GIT* 1991, 10}. However, transition state analogues were not yet employed here for producing selective catalysts.

First successful attempts to produce thermally, chemically and mechanically stable imprints of transition state analogues in the surfaces of inorganic oxides were reported by Morihara *et al*. for the first time in 1988 {K. Morihara, S. Kurihara, J. Suzuki, *Bull. Chem. Soc. Jpn.* 1988, 3991}. The authors showed that it is possible to leave molecular imprints on a silicagel. The catalysts were prepared from silicagel with aluminum chloride by embedding the imprinting molecule. The resulting gel was air-dried at room temperature, and the embedded imprinting molecule which was not chemically bonded was in turn extracted with methanol. The silicagel catalysts thus produced appear to contain cavities complementary to the imprinting molecule, which cavities must contain acidic aluminum atoms as catalytically active sites. Such catalysts were employed for butanolysis of benzoic acid anhydride and exhibit a slight increase in the reaction rate relative to the control catalysts with the use of aluminum chloride, however without imprint. Although with these materials it could be shown that the produced imprints in the silicagel exhibit a slight chemoselectivity and also enantioselectivity {K. Morihara, M. Kurokawa, Y. Kamata, T. Shimada, *J. Chem. Soc. Chem. Commun.* 1992, 358)}, the achievable acceleration and selectivity are not comparable to those provided by enzymes. Also no example of a reaction could be shown which in the absence of the imprints does not proceed at all or proceeds only at an unmeasurable low rate. The relative low selectivity effect, the great susceptibility of the catalyst to becoming poisoned, the unknown thermal and chemical stability and the requirement of working under the exclusion of the humidity of the air and at relatively mild reaction conditions of 55 °C to 60 °C do severely restrict the usability of the Morihara catalyst.

Molecular imprints in inorganic materials are of a high practical interest, since the cavities formed by the imprints enable a selective adsorption to be accomplished of the molecules complementary to the imprints. Such materials can be used in two basically different fields, namely selective adsorption and heterogeneous catalysis.

Selective adsorption may be utilized for the removal of noxious materials, separation of enantiomers, molecular recognition and molecular sensoring. Areas of practiccal application are functions of monitoring air and water, chromatographic separation procdures and separations of racemates. As selective catalysts, these materials can be used for the selective production of organic and inorganic compounds as well as for the selective production of chemical raw materials.

The present invention relates to producing molecular imprints in inorganic materials produced by polymerization of inorganic or organometallic monomer units and to the use of the resulting materials as catalysts. Thermally, mechanically and chemically stable molecular imprints in inorganic solids may be produced during a polymerization process (e.g. a sol-gel process) by including the molecule to be imprinted in the matrix being formed during the polymerization. A particularly efficient incorporation in the inorganic matrix is effected, if the molecule to be imprinted is chemically bond to a monomer unit that can be copolymerized with the inorganic monomer. The removal of the included molecule can be effected by pyrolysis, hydrolysis, photolysis, extraction or oxidative degradation. If the molecules to be imprinted, in their external shape, resemble the transition state or the intermediate of a reaction (hereinbelow designated as transition state analogues), then selective catalysts may be produced in this manner. It was found that such catalysts also catalyze reactions that will not proceed in the absence of the catalyst. On the other hand, analogous reactions are no longer catalyzed, if reactants having a significantly changed structure are employed. Such selective catalysts may also be produced by an organic polymerization in the presence of a suitable transition state analogue, in which case, however, they suffer from problems of a limited thermal stability of the resulting cavities.

The claimed subject matter comprises inorganic solids as catalysts which contain imprints of molecules in the form of cavities, which molecules in their three-dimensional structures conform to intermediates or transition states of chemical reactions to be catalyzed, and processes for producing such solids as specified. Said solids are produced by the polymerization of inorganic or organometal monomer units in the presence of the molecules to be imprinted, wherein the molecules to be imprinted have been chemically bonded to anchor groups of the monomer units and are admixed prior to or in the beginning of the polymerization process. The molecules to be imprinted are removed after the polymerization, so that the imprints in the form of cavities which are characteristic of the selectivity of the catalyst and the anchor groups will remain in the polymer matrix. The essential differences to the Morihara process consist of that
(1) Morihara produces his catalysts by the hydrolysis of silica gels in the presence of the molecules to be imprinted and of aluminum chloride, instead of by polymerization;
(2) in the Morihara procedure the molecules to be imprinted are not chemically incorporated via an anchor group in the polymer matrix and, hence, are thermally sensitive and due to the thermal movement and diffusion processes the cavities produced are less precise than with our procedure.

One embodiment of the process consists of that the inorganic solid is produced by using ortho esters, preferably methyl, ethyl, propyl, butyl or isobutyl esters or mixtures thereof, and preferably (>60%) of the elements Si, Ti, Zr, Al, P, V and additionally (<40%) of La, Mn, Y, Ba, Mg, Pb, Fe, Nb, Sn, Zn or mixtures thereof according to the sol-gel process under neutral, acid, basic or otherwise catalysis. Alternatively suitable solids are metal nitrites and carbides, prepared by wet-chemical polymerization.

The removal of the imprinted molecules upon the completion of the polymerization and optionally after drying and grinding can be effected by evaporation, hydrolysis or decomposition at elevated temperatures or chemically by oxidation or reduction or by employing extractive methods or by thermal, photolytical, hydrolytical or wet-chemical cleavage of the chemical bond to the anchor groups.

The catalysts thus prepared are distinguished in that more than 50% thereof, and predominantly 100% thereof, are amorphous and have a porosity in excess of 5%. They can be heated to at least 250 °C without losing more than 10% of the selectivity thereof.

The catalysts can also be prepared by loading a support material having a large surface area, e.g. a powder of a metal oxide or mixtures thereof, with the catalyst material containing the cavities.

If imprints of several transition state analogues of a multistage reaction are produced in a catalyst material, then said material will be capable of catalyzing several steps of a synthesis.

If the materials will be subsequently loaded with catalytically active sites such as, e.g., sub-group metal oxides or noble metals, they can be employed as supported catalysts, thereby to replace the unselective support materials as conventionally used and to crucially improve the selectivity and activity of heterogeneously catalyzed chemical processes.

Alternatively, a further catalytically active site either can be chemically bonded to the transition state analogue and then be added to the polymerization or can be admixed to the polymerization in addition to the transition state analogue. Suitable as an additional catalytically active site is any atom, cluster or other structure which interactively promotes the catalysis of the desired reaction. These additional sites, if they are present in or near the imprint or were directly bonded to the transition state analogue do not affect the structural selectivity of the reaction which is exclusively controlled by the imprint. If the additional active sites were added only to the inorganic polymerization, then they are capable only of reducing the selectivity since then reactions outside of the imprint will also be catalyzed. Such additional catalytically active sites may be constituted by noble metal atoms, noble metal clusters, non-metals or metal oxides, the object of which is mostly the activation of one of the two reaction components (e.g. H₂, O₂ etc.) or the generation of Lewis or Brønsted acids (incorporation of Al atoms) or basic sites (incorporation of P atoms) on the inorganic surface.

The selective catalysts may be used in all areas of heterogeneous catalysis. Due to the high temperature stability of the molecular imprints, reactions which normally require the presence of a basic, acidic or metallic co-catalyst can be catalyzed exclusively by the selective cavity.

The present invention is suitable for the selective catalysis of intermolecular and intramolecular reactions such as rearrangements, isomerizations, cyclizations, hydrolyses, condensation reactions, exchange reactions, addition reactions, pericyclic reactions, elimination reactions, oxidation and reduction reactions.

If molecules in the ground state are used for the imprint in the place of transition state analogues, then the imprint material formed therewith can be used for molecular recognition. Such materials can be used for a selective adsorption of noxious materials as well as for a selective chromatographic separation. They are suitable for increasing the residence times of volatile molecules on a surface and, thus, for improving the catalytic reaction of such volatile molecules with larger reactants. Such materials may alsoo be employed as molecule-selective sensors in trace analysis by utilizing the change in the physical properties of a thin film of said material by the occupation of the cavities with the target molecule {Y. Yan, T. Bein, in "Synthesis, Characterization and Novel Applications of Molecular Sieve Materials", MRS Symposium Series **233** (1991), page 175; T. Bein, K. Brown, G.C. Frye, J.C. Brinker, *J. Am. Chem. Soc.* **111** (1989) 7641}.

In accordance with the invention, the molecule to be imprinted is bonded by chemical synthesis to one or more suitable monomer units for being incorporated in the inorganic matrix. Then it is added to the monomer mixture for producing the inorganic material and subjected to the polymerization under mild conditions. The attached monomer unit M (anchor) is designed to be chemically incorporated in the inorganic matrix so that with the polymerization proceeding the molecule to be imprinted is completely wrapped by the polymeric inorganic material. Volatile molecules relased in the course of the polymerization such as water, alcohols or other substances are then largely removed from the inorganic structure by mildly and carefully drying, whereupon a solid is formed which largely or exclusively consists of the inorganic polymer and the enclosed imprint molecules (pre-product). This latter material is now dried as mildly as possible and ground. The resulting powder is baked at an elevated temperature so that the included organic molecule is quantitatively evaporated, if possible, or is decomposed, thereby creating the free imprint. Alternatively, the enclosed molecule can be removed from the cavity by way of a mild chemical oxidation, hydrolysis, reduction or of extractive methods. Also, the molecule to be imprinted may be bonded to a monomer unit through a photolabile group, which bond in the pre-product is then cleft by irradiation, whereby the imprint molecule can be more readily removed. Once the imprint molecule has been removed as completely as possible, the finished catalysator comprising the free selective cavities is available. In said cavities there are preferably adsorbed the original model molecule or molecules having similar structural features which, due to the size thereof, can fit into the bonding site thus provided.

If a molecule which resembles a transition state or an intermediate of a reaction is used for forming the imprint, then the imprint causes a stabilization of the transition state or the intermediate of the reaction and, hence, an acceleration of the reaction. In this case the cavity is a selective catalyst for the reaction. Such a catalyst basically is usable without limitations. After any possible deactivation it can be reactivated by another baking step. 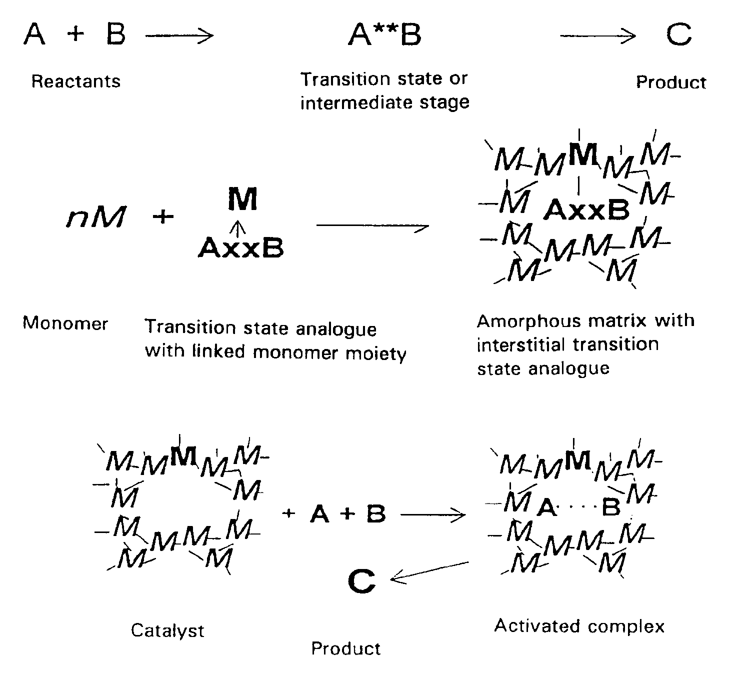
In this manner, selective catalysts are preparable for any reaction, if only a transition state analogue or an intermediate compound analogue can be synthesized and can be linked to a monomer unit of the target matrix. If, in order to accelerate the desired reaction, a co-catalyst such as, for example, a noble metal atom is attached in the proximity of or within the selective cavity, then the selectivity and activity of the reaction catalyzed by the co-catalyst should be significantly improved.

In this way, in principle, the selectivity of of all known chemical reactions can be enhanced. However, in addition even reactions that so far have not been realizable can be enforced, if the desired reaction as normally not occurring is favored by a suitable selective cavity.

If a thermally, chemically and mechanically stable material is used as the inorganic matrix of this catalyst, then in this way selective catalysts can be produced which can be employed at reaction temperatures of up to a maximum of 900 °C, if the amorphous structure of the material can be retained at such temperatures. The most important pre-requisite of the functionability of the catalyst is the generation of an amorphous structure, since only such an amorphous structure is capable of providing a sufficiently accurate accomodation to any optional imprint molecule. The formation of crystalline regions destroys the capability for a molecular recognition and, thereby, the selectivity.

### EXAMPLE 1a

Preparation of a selective catalyst (I) from pure silicon dioxide for a transesterification of phenylacetic acid ethyl ester with n-hexanol to form phenylacetic acid hexyl ester 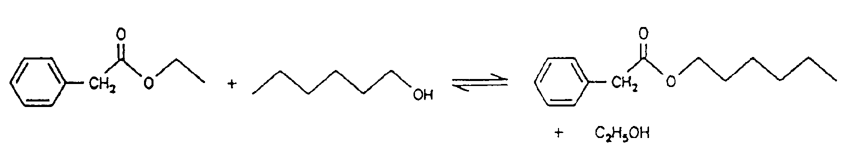
The captioned reaction will normally proceed at a measurable rate of reaction via transition states or reaction intermediates a through c only with acid or base catalysis. 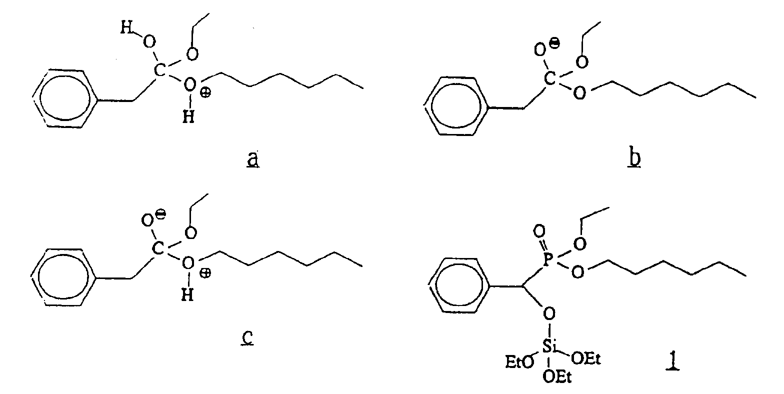
As the transition state analogue there was synthesized the phosphonate **1** which was linked to a triethoxysilyloxy moiety as the monomer unit. The synthesis was started with the commercially available diethyl phosphite which was converted into the monosodium salt with NaOH. Substitution of the tetrabutylammonium cation for the Na ion and reaction with n-hexyl iodide afforded the mixed ethyl hexyl phosphite. The latter compound was coupled with benzaldehyde in the presence of CsI to form 1-hydroxybenzyl phosphorous acid hexyl ethyl ester, which was then reacted with pyridine and chlorotriethoxysilane to form the transition state analogue with the monomer unit linked thereto.

Silicon dioxide as the catalyst was chosen, because, as an amorphous inorganic solid, it is catalytically inactive itself. It is shown that the catalyst I catalyzes the transesterification under conditions, at which the reaction fails to proceed both in the absence of a catalyst and in the presence of the control silicon dioxide II.

### Preparation of the catalyst I:

Fifty-five ml of tetraethoxysilane, 50 ml of ethanol and 9 ml of 8 N HCl were filtrated through an aluminum oxide membrane, pore size of 10 nm, in order to remove dust, germs and oligomers. 27.5 ml of Si(OEt)₄ and 231 mg of the above phosphonate **1** were charged into a 100 ml polypropylene beaker and, while stirred, were slowly admixed first with 25 ml of EtOH and then with 4.5 ml of the hydrochloric acid, whereby the sample became warmed up to 50 °C. The warmed solution was allowed to sit uncovered under a fumehood for 4 days. In the course thereof a brittle glassy solid (pre-product) was formed. The solid was ground to form a fine powder and was heated at a heating rate of 1.5 °C per minute to 250 °C, maintained at that temperature for 5 hours and then cooled to room temperature at a cooling rate of about 2.5 °C/min, whereupon the ready-to-use catalyst I was obtained. By accurately the same procedure, except for the absence of the transition state analogue 1, control silica I was prepared from the remaining filtrated substances.

Analytical examinations: Investigations with a high-resolution electron microscope showed that the catalyst was completely amorphous and consisted of pure silicon dioxide. By means of solid state NMR it could be determined that the transition state analogue was firmly incorporated in the pre-product and not present just in an adsorbed state, while in the final catalyst the transition state analogue was not detectable any more. This finding was confirmed by temperature-dependent mass spectrometry, wherein the monomer-free transition state analogue could be released from the pre-product, whereas the final catalyst as well as the control product did not continue to release any desorption products.

Ten mmoles of phenylacetic acid ethyl ester and 100 mmoles of n-hexanol (both freshly filtrated through aluminum oxide, neutral, in order to remove any traces of acid), when mixed, did not exhibit any detectable reaction at 140 °C over a period of 2 hours; after 2 hours, autocatalysis became noticeable. In the presence of 1 g of the Control Silica Ia no reaction was detectable over 48 hours. Using 1 g of the Catalyst I, an immediate conversion was observed, proceeding at a rate of reaction of 0.07 mmoles of ester per hour and per gram of catalyst. Hereby it was shown that the reaction, which fails to proceed in the presence of the cavity-free SiO₂ under these conditions, is catalyzed in the presence of the cavity.

### COMPARATIVE EXAMPLE 1b:

In I, the cavity produced acts according to the principle of molecular recognition. The original model substrate is recognized, and a transesterification is catalyzed only of compounds which are similar to the original substrates.

In the same manner as in Example 1a, 5 mmoles of 1-naphthylacetic acid ester and 50 mmoles of 2-phenylethanol were heated at 140 °C in the presence of 1 g of catalyst, without any reaction being observable. After the reaction mixture had been maintained at 140 °C for 12 hours, 5 mmoles of phenylacetic acid ethyl ester and 50 mmoles of n-hexanol were added thereto. The formation of phenylacetic acid hexyl ester was detectable already after 2 hours, whereas of the three other possible transesterification products not even traces were recognizable. The experiment demonstrates the substrate selectivity of the catalyst. Obviously, the catalyst refuses to transesterify any of the substrates naphthylacetic acid ester and 2-phenyl ethyl alcohol which are different from the original model reaction.

### COMPARATIVE EXAMPLE 1c:

In I, the cavity produced acts according to the principle of molecular recognition, that is, it recognizes the original model substrate and refuses to catalyze a transesterification even if the original ester is employed and only the alcohol to be transesterified exhibits some higher structural difference from the model alcohol.

In the same manner as in Example 1a, 10 mmoles of phenylacetic acid ethyl ester were reacted with an 1:1:1 mixture of 100 mmoles of n-octanol, 100 mmoles of n-hexanol and 100 mmol of 2-phenylethanol in the absence of a catalyst (autocatalysis). All of the three esters possible to be newly formed were produced at comparative rates (unselectively). This experiment was repeated using 1 g of the Control Silica Ia. After 24 hours, any transesterification product was not yet detectable. The repetition of this experiment using 1 g of the Catalyst I showed that n-octanol and n-hexanol underwent the reaction at comparable rates, whereas no reaction was detectable with phenylethanol. This experiment demonstrates the high substrate selectivity of the catalyst. The catalyst obviously tolerates octanol in the place of hexanol, whereas it already refuses to transesterify the intial phenylacetic acid ethyl ester with 2-phenylethanol which, upon comparison, is structurally different from n-hexanol.

### COMPARATIVE EXAMPLE 1d:

The cavities in I do not only act as a catalyst. They do also act as a co-catalyst. To 100 mmoles of phenylacetic acid ethyl ester and 100 mmoles of n-hexanol in 60 ml of boiling absolute toluene under an argon blanket there were added with stirring 100.1 mg of concentrated sulfuric acid. Here the rate of reaction of the formation of phenylacetic acid hexyl ester was 18 ± 1 mmoles/hour per gram of sulfuric acid. The reaction carried out in the same manner, however with the addition of 400 mg of Catalyst I, exhibited an acceleration to 27 ± 1 mmoles/hour per gram of sulfuric acid. When an analogous reaction was carried out in the absence of a catalyst, but in the presence of 400 mg of Comparative Silicon Dioxide II, the reaction rate decreased to 10 mmoles/hour per gram of sulfuric acid. Thus, the acid-catalyzed transesterification of phenylacetic acid ethyl ester with n-hexanol at 110 °C is accelerated by the addition of I. Relative to the Control Silica Ia, this is an acceleration by a factor of 2 to 3. In an analogous experiment, I does not accelerate the transesterification of naphthylacetic acid ethyl ester, but it exerts the same slightly inhibitive action on this reaction as the Control material Ia does. Also this investigation confirms that the Silicon Dioxide I contains active sites for the catalysis of the target reaction.

### COMPARATIVE EXAMPLE 1e:

Since the tested reaction is capable of proceeding autocatalytically, there remained to be examined whether or not the observed reaction behavior of the Catalyst I would be attributable to a decomposition of the transition state analogue to form phosphorous acid on the silicon dioxide. To this end, the Control Silica Ia (450 mg) was loaded with 50 mg of phosphorous acid, mixed with 100 mmoles of n-hexanol, 100 mmoles of octanol, 100 mmoles of 2-phenylethanol and 10 mmoles of phenylacetic acid ethyl ester, and the mixture was stirred and heated to 150 °C. While Catalyst I did not catalyse the transesterification with phenylethanol, the acidic silicon dioxide employed here was absolutely unselective, all of the three esters possible to be newly formed were produced at the same rate. Thereby it was shown that the selectivity as observed of I is not caused by any adsorption effects or other surface interactions of the various substrates on the microporous silicon dioxide.

### EXAMPLE 2:

In this example it is demonstrated that cavities usable for selective catalysis can be produced also in mixed metal oxides in accordance with the process presented here. It was expected that a monomer mixture comporising several metal oxides, due to the increased structural variety of the monomers, would allow an improved accomodation to the transition state analogue of the cavity to be formed, which ought to result in an improved catalyst activity.

In the same manner as in Example 1a, a catalyst II was prepared from a mixture comprising 25 ml of EtOH, 12.5 ml of Si(OEt)₄, Zr(O-nPr)₄, 5 ml of Ti(O-npr)₄, 5 ml of Al(O-sBu)₃, 4.5 ml of 8 N HCl and 308 mg of phosphite 1. 100 moles of n-hexanol were allowed to react with 10 mmoles of phenylacetic acid ethyl ester and 1 g of the resulting catalyst at 140 °C. This catalyst showed a rate of reaction of 0.77 mmoles/h·g, which activity is 10 times higher than that of Catalyst I made of pure SiO₂. This experiment demonstrates that catalysts made of mixed metal oxides can as well employed, like the pure SiO₂ catalysts.

### EXAMPLE 3:

Preparation of a selective catalyst by copolymerization of tetraethoxysilane with phenyltriethoxysilane, methyltriethoxysilane and a transition state analogue.

In this example it is demonstrated, that cavities suitable for selective catalysis can also be produced in mixed organic-inorganic metal oxides.

90 mmol Tetraethoxysilane, 10 mmol triethoxymethylsilane, 10 mmol triethoxyphenylsilane, 484 mg phosphonate 1 were dissolved in 400 mmol ethanol at room temperature. To this solution, 10 mg ammonium fluoride, dissolved in 4 ml water, were added at once. After 10 min, gelation was observable. The gel was then dried by heating to 102 °C with a heating rate of 0.1 °C/min, kept at 120 °C for 6 hours, and then heated to 250 °C with a heating rate of 0.5 °C/min and kept at 250 °C for 6 hrs. The material was then cooled to room temperature with a cooling rate of 1.5 °C. The powdered catalyst III catalyzed the transesterification of phenylacetic ethylester to the hexylester. Although selective, this catalyst was less selective then the pure silica catalyst I described in example 1.

This example shows, that selective catalysts can also be prepared by the copolymerization of orthoesters with organoalkoxides with fluoride instead of acid catalysis.

### EXAMPLE 4

Preparation of a selective silica-catalyst VI for the formation of lactones from hydroxyesters. The catalyst is here used to promote the disfavored reaction and suppress the normally favored polymerization reaction: 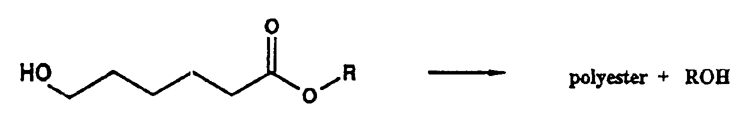
disfavored reaction: 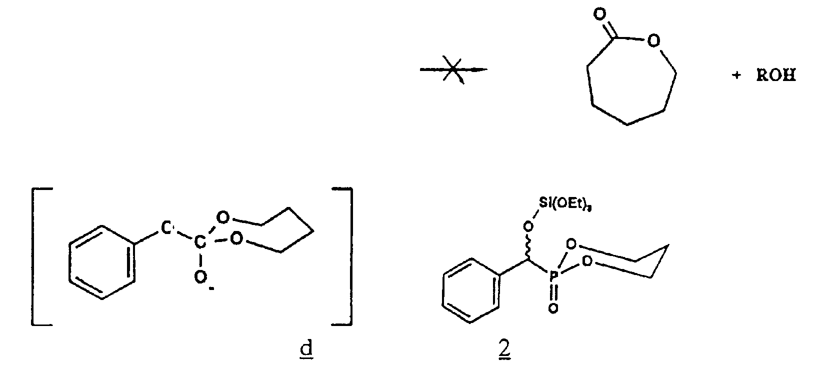
This reaction in the presence of acid or base catalysts results in the formation of polyesters. The disfavored formation of the seven-membered lactone can be formulated to proceed through the intermediate d. As transition state analogue the phosphonate 2 was synthesized: 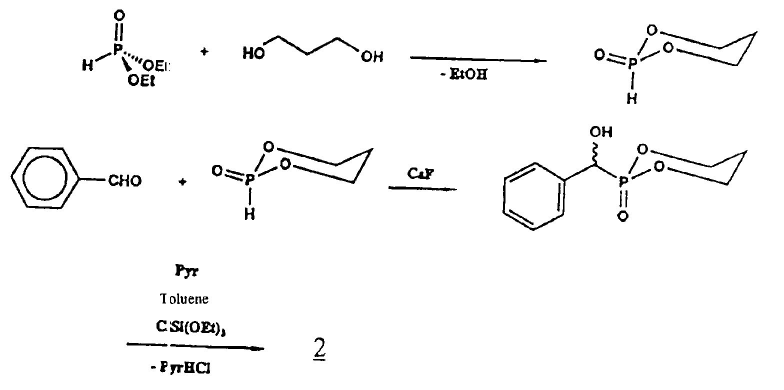
As in example 1a, the selective catalyst was prepared by copolymerization of 2 with tetraethoxysilane, followed by drying and calcination.

14 ml Tetraethoxysilane, 12.5 ml ethanol, 2.5 ml 0.4 N HCl, filtrated through an aluminum oxide membrane, pore size 10 nm, in order to remove dust, germs and oligomers and 330 mg of the phophonate 2 were charged into a 100 ml polypropylene beaker and stirred for 4 days. In the course thereof a brittle glassy solid (pre-product) was formed. The solid was ground to form a fine powder and was heated at a heating rate of 0.5 °C per minute to 250 °C, maintained at that temperature for 6 hours and then cooled to room temperature at a cooling rate of about 1.5 °C/min, whereupon the ready-to-use catalyst VI was obtained. By accurately the same procedure, except for the absence of the transition state analogue **2**, a control material VIa was prepared.

To 149 mg catalyst powder in 2.5 g m-xylene at 140 °C a solution of 104 mg phenyl-6-hydroxyheptanoate, dissolved in 0.5 g xylene, were added under stirring. At a reaction temperature of 140 °C the reaction was monitored by gas chromatography. The only products detectable were the 6-methyl-ε-lactone and phenol. The 1:1-formation of the two products is indicative of selective lactone formation. No polyester formation was detectable. The same reaction carried out in the presence of acid in the absence of the silica catalyst resulted in the formation of polyester as the only product, while the control silica VIa without imprints showed no activity.

This example shows, that imprinted silica catalysts can be used to selectively enhance disfavored reactions, like the formation of seven-membered ring compounds.

### EXAMPLE 5

Preparation of a selective silica catalyst for the cycloaddition of butadiene and norbornadiene: 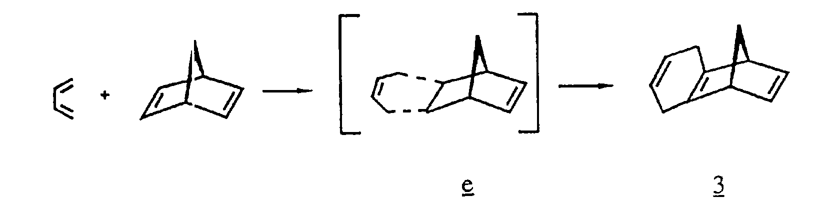
The transition state analogue 4 has approximately the same spacial extension as the transition state e. 4 was synthesized by the procedure shown below:

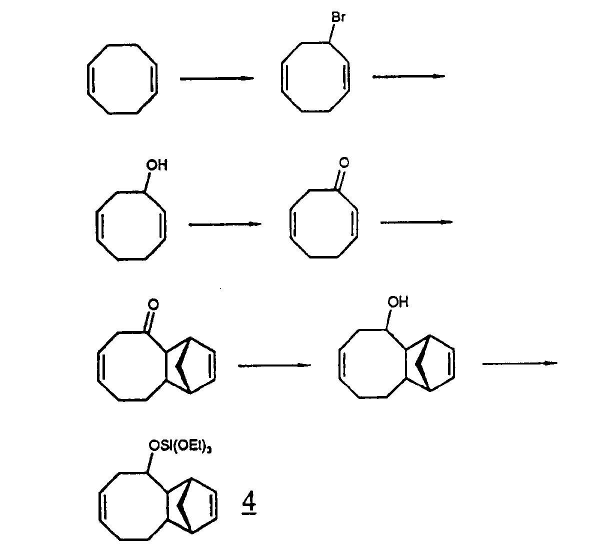

4 + n (EtO)₄Si + m Me(EtO)₃Si + H₂O + H⁺ --> 4-(SiO₂)ₙ-(MeSiO_{3/2})ₘ

The catalyst was prepared by copolymerization of 4 with tetraethoxysilane and methyltriethoxysilane. 4.75 ml tetraethoxysilane, 0.75 ml methyltriethoxysilane and 0.5 ml 4 were added to a solution of 2.5 ml 0.4 n hydrochloric acid in 5 ml ethanol at 55 °C. The solution was kept at this temperature for 24 h. The resulting cracked glass catalyst V was heated to 65 °C at a rate of 1 °C/min, kept there for 5 h and then heated to 250 °C at a rate of 0.1 °C/min. After keeping the material for 5 h at 250 °C, it was allowed to cool to room temperature.

190 mg of the above catalyst V were ground in a ball mill and put into a clean autoclave under Ar. At a temperature of -22 °C 5 ml butadiene and 1 m norbornadiene were added. The autoclave was sealed and heated to 80 °C. After 5 days the autoclave was opened and a conversion of 45% of the norbornadiene to the tricyclo[4.4^{1,6}.2^{2,5}.1^{2,5}]undeca-3,8-diene (3) was observed.

A control experiment without the silica catalyst showed no product formation.

This example shows, that imprinted silica catalysts can be used to catalyze cycloaddition reactions.

### EXAMPLE 6

Preparation of a selective adsorbent from silica for the adsorption of (-)-2-borneol.
compound used for imprinting: 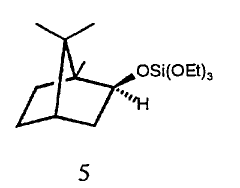
In a 100 ml PE-beaker 45 mmol tetraethoxysilane, 5 mmol methyltriethoxysilane and 2.5 mmol bornyloxytriethoxysilane 5 were dissolved in 10 ml ethanol and stirred for 5 min at room temperature. 5 ml 0.4 N hydrochloric acid were added at once and the mixture was heated to 55 °C for 48 hrs. without stirring. The cracked glass was cooled to room temperature and then heated to 65 °C with a heating rate of 0.1 °C/min, kept at this temperature for 5 hrs., heated to 250 °C at the same heating rate of 0.1 °C/min and kept at 250 °C for another 5 hrs. The dried and activated glass IV was cooled to room temperature at a cooling rate of 2 °C/min, ground for 30 min in a ball mill and kept under a vacuum < 0.1 torr for 5 hrs.

A solution of 613 mg (-)-borneol, 62.4 mg (+)-fenchol and 61 mg camphor in 25 ml decylbenzene was prepared as standard. 200 mg of the calcined glass IV were added to 2 ml of the standard solution and stirred in a closed flask. The composition of the solution was monitored by calibrated gas chromatography. Already after 25 min, 60% of the (-)-borneol were adsorbed selectively, while the concentration of the other two components remained constant. After 215 min, 75% of the borneol was adsorbed.

This example shows, that a very high substrate specificity for adsorption can be obtained by molecular imprinting in amorphous metal oxides.

### EXAMPLE 7:

Enantioselective adsorption of (-)-borneol
200 mg of imprinted glass IV were stirred with 2 ml of a solution of 600 mg (-)-borneol and 600 mg (+)-borneol in 125 ml decylbenzene in a closed flask. The change in composition with time was monitored by gas chromatography with a chiral capillary column. After 150 hrs. the concentration of the (-)-borneol was reduced by 10%, while the concentration of the (+)-borneol remained constant documenting enantioselecitve adsorption.

This example shows, that enantioselective adsorption can be observed by chiral molecular imprinting in amorphous metal oxides.

## Claims

1. A process for producing inorganic solids as catalysts which contain imprints of molecules in the form of cavities, which molecules in their three-dimensional structures conform to intermediates or transition states of chemical reactions to be catalyzed, characterized in that inorganic or organometallic monomer units are polymerized in the presence of the molecules to be imprinted, wherein the molecules to be imprinted have been chemically bonded to copolymerizable anchor groups (monomer units) and are admixed prior to or in the beginning of the polymerization process and the imprinted molecules are subsequently removed after the polymerization.

2. The process according to claim 1, characterized in that the inorganic solid is produced by using ortho esters, preferably with R = methyl, ethyl, propyl, butyl or isobutyl alkoxides or mixed alkoxides having the general structure of (RO)M or (RO)ₓMR'_{y} with y = 0-2 and R' being any optional organic moiety, or mixtures thereof, and M being preferably (>60%) the elements Si, Ti, Zr, Al, P, V and additionally (<40%) La, Mn, Y, Ba, Mg, Pb, Fe, Nb, Sn, Zn or mixtures thereof according to the sol-gel process under neutral, acid, basic or otherwise catalysis.

3. The process according to claim 1, characterized in that the inorganic solid is a metal nitrite or carbide which is prepared by wet-chemical polymerization.

4. The process according to claim 1, characterized in that the removal of the imprinted molecules after the completion of the polymerization and, if desired, after drying and grinding, is effected by evaporation or decomposition at elevated temperatures or chemically by oxidation or reduction or by means of extractive methods.

5. The process according to claim 1, characterized in that the removal of the imprinted molecules is effected by thermal, photolytic, hydrolytic or wet-chemical cleavage of the chemical bond to the anchor groups.

6. Inorganic solids as catalysts containing imprints of molecules in the form of cavities which molecules in their three-dimensional structures conform to intermediates or transition states of chemical reactions to be catalyzed.

7. Inorganic solids as catalysts, characterized in that they have been produced according to any of claims 1 through 5.

8. A catalyst according to claim 7, characterized in that more than 50% thereof, and preferably 100% thereof, are amorphous and it has a high (>5%) porosity.

9. A co-catalyst according to claim 7, which co-catalyst additionally accelerates a reaction catalyzed by a primary catalyst in the form of an acid or a base or a transition metal or a transition metal complex.

10. A catalyst according to claim 7, characterized in that the catalyst contains a catalytically active site in the form of an acid, a base or a transition metal, in addition to the imprints of intermediate or transition state analogues, said site having been linked to the molecule to be imprinted via one or more chemical bond(s) and in this state has been added to the polymerization or said site, in the form of a soluble molecule, having been admixed to the polymerization batch in addition to the molecule to be imprinted, while said additional catalytically active site, upon the removal of the imprinted molecule from the solid polymerization product, remains in or on the matrix solid within the cavity or in the proximity thereof.

11. A catalyst according to claim 7, characterized in that the imprints may have originated from several different chemical compounds corresponding to intermediate stages or transition states of a multistage catalytic synthesis.

12. A catalyst according to claim 7, characterized in that more than 10% of the molecules to be imprinted have been removed from the inorganic solid.

13. A catalyst according to claim 7, characterized in that it can be heated to at least 250 °C without losing more than 10% of its selectivity.

14. A catalyst, produced according to any of claims 1 to 2, characterized in that the polymerization is carried out in the presence of a support material having a large surface area, and preferably a metal oxide powder or mixtures thereof, and that after the production of the catalyst the surface of the support material has been occupied by a thin layer of the cavity-containing catalyst.

15. Use of the catalysts according to any of claims 7 to 15 for the selective catalysis of reactions wherein molecules which, in their three-dimensional structure, correspond to the molecules imprinted in the cavities of the catalyst appear as intermediates or transition states.
